**Europäisches Patentamt**

**European Patent Office**

**Office européen des brevets**

(19)

(11) Veröffentlichungsnummer: **0 189 547 B1**

# (12) EUROPÄISCHE PATENTSCHRIFT

(45) Veröffentlichungstag der Patentschrift:
27.09.89

(51) Int. Cl.⁴: **C 07 D 307/89**

(21) Anmeldenummer: **85115042.5**

(22) Anmeldetag: **27.11.85**

(54) Verfahren zur Entfernung von Naphthochinon aus Phthalsäureanhydrid.

(30) Priorität: **26.01.85 DE 3502682**

(43) Veröffentlichungstag der Anmeldung:
**06.08.86 Patentblatt 86/32**

(45) Bekanntmachung des Hinweises auf die Patenterteilung:
**27.09.89 Patentblatt 89/39**

(84) Benannte Vertragsstaaten:
**AT BE DE FR GB IT NL SE**

(56) Entgegenhaltungen:
**EP-A- 0 120 199**
**US-A- 2 510 852**
**US-A- 3 462 461**

**Die Akte enthält technische Angaben, die nach dem Eingang der Anmeldung eingereicht wurden und die nicht in dieser Patentschrift enthalten sind.**

(73) Patentinhaber: **HÜLS AKTIENGESELLSCHAFT, Patentabteilung / PB 15 - Postfach 13 20, D-4370 Marl 1 (DE)**

(72) Erfinder: **Gude, Fritz, Dr., Zur Emschermulde 24, D-4690 Herne 2 (DE)**
Erfinder: **Scharf, Helmut, Dr., Kilianstrasse 75, D-4235 Schermbeck (DE)**

EP 0 189 547 B1

## Beschreibung

Bei der Herstellung von Phthalsäureanhydrid durch Luftoxidation von Naphthalin und Naphthalin enthaltenden Gemischen in der Gasphase fällt ein Rohphthalsäureanhydrid an, das durch Naphthochinon verunreinigt ist. Während die meisten Nebenprodukte, wie z.B. harzartige Verbindungen, Maleinsäureanhydrid und Schwefel leicht durch Destillation abgetrennt werden können, verbleibt Naphthochinon, dessen Siedepunkt im Siedebereich des Phthalsäureanhydrids liegt, bei der Destillation im Phthalsäureanhydrid und vermindert dessen Farb- und Reinheitsqualität. Daher ist es erforderlich, das Naphthochinon vor der Destillation des Rohphthalsäureanhydrids möglichst weitgehend zu entfernen, wobei beim raffinierten Rohprodukt Werte von ⩽ 5 ppm Naphthochinon und beim destillierten Phthalsäureanhydrid maximal 1 ppm Naphthochinon angestrebt werden sollten.

Die Entfernung des Naphthochinons erfolgt in der Regel durch Umwandlung des Naphthochinons in höher kondensierte Produkte, die dann bei der anschliessenden Destillation des Rohphthalsäureanhydrids im Rückstand verbleiben. Als bevorzugten Katalysator für die Konversion wird häufig Schwefelsäure bei hohen Temperaturen (z.B. Ullmann's Encyclopädie der Technischen Chemie, 3. Auflage, Vol. 13, S. 723) oder auch z.B. ein Gemisch aus Schwefelsäure und Borsäure (US-PS 2 850 440) vorgeschlagen. Die Verwendung von Schwefelsäure führt jedoch zu Korrosionsproblemen, und die entstehenden sauren Abgase sowie die bei der Neutralisation der Schwefelsäure (und gegebenenfalls Borsäure) entstehenden Salze bringen erhebliche Probleme bei der Destillation des Rohphthalsäureanhydrids mit sich. Neben den stark sauren Katalysatoren sind auch alkalische Katalysatoren, wie z.B. Alkalihydroxide (z.B. US-PS 2 670 325, US-PS 4 165 324) und Natriumcarbonat/Zinn-(II)-chlorid (US-PS 2 356 499) empfohlen worden. Alkalische Katalysatoren haben aber den Nachteil, dass sie einen Teil der Phthalsäure decarboxylieren und so zu einem Verlust an Phthalsäureanhydrid beitragen. Ausserdem können grössere Mengen Alkalihydroxid zu einer explosionsartigen Polymerisation der im Rohphthalsäureanhydrid enthaltenen Maleinsäure führen. Andere für die Raffination von Rohphthalsäure vorgeschlagene Substanzen sind z.B. Kaliumhydrogensulfit und Kaliumpyrosulfat (JA-PA 10323/1970). Der Nachteil dieser Verbindungen ist, dass der bei der Destillation des raffinierten Rohphthalsäureanhydrids anfallende Rückstand grössere Mengen an Schwefel enthält, der bei der Beseitigung des Rückstandes, z.B. durch Verbrennen, zu Umweltbelastungen führt.

Gemäss EP-PS 120 199 erfolgt die Entfernung des Naphthochinons mittels Polybutadienöl, einem acyclischen ungesättigten Kohlenwasserstoff, insbesondere in Gegenwart von Alkaliionen. Das Verfahren der US-PS 2 510 582 verwendet Maleinsäureanhydrid, also eine heterocyclische Verbindung zur Entfernung des Naphthochinons. Schliesslich benennt US-PS 3 462 461 u.a. für den Zweck mehrfach ungesättigte Kohlenwasserstoffe, die wenigstens 2 olefinische Doppelbindungen in einer acyclischen Kette besitzen.

Aufgabe der vorliegenden Erfindung war es, für das bei der Herstellung von Phthalsäureanhydrid durch Luftoxidation von Naphthalin oder Naphthalin enthaltenden Gemischen anfallende Rohphthalsäureanhydrid eine Raffinationsmethode zu entwickeln, die es ermöglicht, den Naphthochinon-Gehalt im Rohphthalsäureanhydrid von ca. 0,2 – 2 Gew.-% auf ⩽ 5 ppm und im destillierten Reinphthalsäureanhydrid auf maximal 1 ppm zu reduzieren, die den anschliessenden Destillationsvorgang nicht stört und die zu einem nicht mit zusätzlichen Schwefelverbindungen belasteten, leicht zu beseitigenden Destillationsrückstand führt.

Diese Aufgabe wird erfindungsgemäss dadurch gelöst, dass das bei der Luft-Oxidation von Naphthalin oder Naphthalin enthaltenden Gemischen anfallende Rohphthalsäureanhydrid mit 1,5,9-Cyclododecatrien und/oder seinem Destillationsrückstand bei höheren Temperaturen einige Stunden lang behandelt wird. Bei diesem Raffinationsvorgang wird der Gehalt des in dem Rohphthalsäureanhydrid vorhandenen Naphthochinons von bis zu 2 Gew.-% auf ⩽ ppm reduziert, so dass bei der anschliessenden Destillation ein Reinphthalsäureanhydrid erhalten wird, dessen Naphthochinon-Gehalt maximal 1 ppm beträgt.

Gegenstand der Erfindung ist daher ein Verfahren zur Entfernung von Naphthochinon aus durch Luftoxidation von Naphthalin oder Naphthalin enthaltenden Gemischen hergestelltem Phthalsäureanhydrid, dadurch gekennzeichnet, dass das Rohphthalsäureanhydrid mit 1,5,9-Cyclododecatrien und/oder dessen Destillationsrückstand bei höheren Temperaturen behandelt wird.

Das 1,5,9-Cyclododecatrien stellt man technisch durch Cyclotrimerisation von Butadien her und reinigt es durch Vakuumdestillation. Der Destillationsrückstand enthält neben Cyclododecatrien noch im wesentlichen Oligomere des Butadiens, nämlich $(C_4H_6)_n$, wobei n = 4 – 8, d.h. Oligomere von $C_{16} - C_{32}$, ist. Das erfindungsgemässe Verfahren hat den Vorteil, dass der Raffinationsprozess sehr effektiv ist, so dass die gewünschten hohen Reinheitsanforderungen an das Produkt erfüllt werden können, und dass als Raffinationssubstanz reine Kohlenwasserstoffe verwendet werden, die den Destillationsvorgang bei der Destillation des Rohphthalsäureanhydrids nicht stören, im Reinphthalsäureanhydrid nicht vorhanden sind und einen Destillationsrückstand ergeben, der ohne Probleme umweltfreundlich beseitigt werden kann.

Bei dem erfindungsgemässen Verfahren wird das Naphthochinon enthaltende Rohphthalsäureanhydrid mit einer kleinen Menge 1,5,9-Cyclododecatrien und/oder seinem Destillationsrückstand und gegebenenfalls einer geringen Menge Alkaliionen erhitzt. In dem so behandelten Rohphtalsäureanhydrid ist die Konzentration an Naphthochinon so weit reduziert, in der Regel ≤ 5 ppm, dass das nach der Destillation des Rohphthalsäureanhydrids erhaltene Reinphthalsäureanhydrid i.a. einen Schmelzpunkt von 131,2 °C, einen Naphthochinon-Gehalt ≤ 1 ppm und eine Farbzahl < 10 APHA besitzt. Die Konzentration an Naphthochinon in dem Rohphthalsäureanhydrid, das durch Gasphaseoxidation von Naphthalin oder Naphthalin enthaltenden Gemischen, wie z.B. Naphthalin-o-Xylol-Gemischen, ist für das erfindungsgemässe Verfahren nicht kritisch. Geeignet sind alle gängigen, bei der Herstellung von Phthalsäureanhydrid nach dem Gasphaseverfahren anfallenden Rohprodukte. Der Naphthochinon-Gehalt dieser Produkte liegt je nach dem verwendeten Katalysator und den Betriebsbedingungen bei der Herstellung von Phthalsäureanhydrid in der Regel zwischen ca. 0,2 und ca. 2 Gew.-% und der Gehalt an Maleinsäureanhydrid zwischen 0,01 und 2 Gew.-%.

Das für die Bindung des Naphthochinons verwendete 1,5,9-Cyclododecatrien ist handelsüblich, und der Destillationsrückstand fällt bei der Reinigung des 1,5,9-Cyclododecatriens an. Die für die Raffination des Rohphthalsäureanhydrids benötigten Mengen an Cyclododecatrien oder an Destillationsrückstand oder an Gemischen aus Cyclododecatrien und Destillationsrückstand betragen ca. 0,01 bis ca. 1 Gew.-%, bezogen auf Rohphthalsäureanhydrid. Es sind natürlich auch kleinere und grössere Mengen an Raffinationsmittel möglich, jedoch bringen sie keine Vorteile mehr. Bevorzugt werden Mengen von ca. 0,05 bis 0,5 Gew.-%. Bei Naphthochinon-Gehalten im Rohphthalsäureanhydrid, die wesentlich grösser als 1,5 Gew.-% sind, muss auch der Anteil an Raffinationsmittel entsprechend erhöht werden.

Für die Effektivität der Raffination hat es sich als zweckmässig erwiesen, neben dem Cyclododecatrien und/oder seinem Destillationsrückstand auch Alkaliionen zuzugeben. Als Ionen werden Natrium, Kalium und Lithium bevorzugt. Die an Alkaliionen benötigte Menge beträgt in der Regel 1 bis 40 ppm, bezogen auf die Menge an Rohphthalsäureanhydrid, und besonders bevorzugt werden Mengen zwischen 2 und 20 ppm.

Die Behandlung des Rohphthalsäureanhydrids mit Cyclododecatrien und/oder dem Destillationsrückstand und gegebenenfalls Alkaliionen erfolgt bei Temperaturen zwischen ca. 180 °C und ca. 280 °C, bevorzugt werden Temperaturen im Bereich von 220 °C – 270 °C und die Dauer der Behandlung beträgt ca. 4 h bis 20 h. Es sind aber auch längere Zeiten möglich, jedoch bringen sie im allgemeinen keine Vorteile mehr. In der Regel richtet sich die Behandlungsdauer nach der benötigten Zeit für die anschliessende Destillation.

Anhand der folgenden Beispiele wird das erfindungsgemässe Verfahren weiter verdeutlicht.

### Beispiel 1

500 g Rohphthalsäureanhydrid, das durch Gasphasenoxidation von Naphthalin gewonnen wurde und einen Gehalt an Naphthochinon (NCh) von 0,04 Gew.-% und an Maleinsäureanhydrid (MSA) von 0,03 Gew.-% aufwies, wurden mit 0,3 Gew.-% 1,5,9-Cyclododecatrien (CDT) und 30 ppm wasserfreie Soda (13 ppm Na$^+$) bei 270 °C 7 h lang behandelt. Nach dieser Raffination lag der Naphthochinon-Gehalt im Produkt bei 1 ppm und nach der anschliessenden Kolonnendestillation im Vakuum bei 0,1 ppm.

### Beispiel 2

Beispiel 1 wurde wiederholt mit einem Produkt, das 0,5 Gew.-% NCh und 0,04 Gew.-% MSA enthielt. Nach der Behandlung mit CDT betrug der NCh-Gehalt 1 ppm und nach der Destillation 0,1 ppm.

### Beispiel 3

Beispiel 1 wurde wiederholt mit einem Rohprodukt, dessen NCh-Gehalt 1,5 Gew.-% und dessen Gehalt an MSA 0,03 Gew.-% betrug. Der NCh-Gehalt lag nach der Raffination bei 1 ppm und nach der Destillation bei 0,5 ppm.

### Beispiel 4

500 g Rohphthalsäureanhydrid mit einem NCh-Gehalt von 0,2 Gew.-% und einem Gehalt von 0,03 Gew.-% MSA wurde 7 h bei 270 °C mit 0,4 Gew.-% CDT und 10 ppm wasserfreie Soda (4,3 ppm Na$^+$) intensiv gerührt. Danach war die NCh-Konzentration auf 1 ppm gefallen. Nach der sich anschliessenden Destillation enthielt das Reinprodukt nur noch 0,1 ppm NCh.

### Beispiel 5

Ein Rohphthalsäureanhydrid, das 0,08 Gew.-% MSA und 0,2 Gew.-% NCh enthielt, wurde 7 h bei 260 °C mit 0,05 Gew.-% CDT und 15 ppm Ätzkali (10,4 ppm K$^+$) kräftig gerührt. Nach dieser Behandlung war der NCh-Gehalt auf 4 ppm gesunken. Die anschliessende Kolonnendestillation ergab ein Reinphthalsäureanhydrid mit einem NCh-Restgehalt von 1 ppm.

### Beispiel 6

Ein Rohphthalsäureanhydrid, enthaltend 0,4 Gew.-% MSA und 0,9 Gew.-% NCh wurde mit 0,6 Gew.-% CDT und 40 ppm Ätznatron (23 ppm Na$^+$) versetzt und 6 h unter intensivem Rühren auf 250 °C erhitzt. Danach war der NCh-Gehalt auf 2 ppm gesunken. Eine Feindestillation ergab dann ein Produkt mit 0,5 ppm NCh.

### Beispiel 7

Ein Rohphthalsäureanhydrid enthielt 1,5 Gew.-% NCh und 0,03 Gew.-% MSA. Es wurde mit 0,4 Gew.-% CDT und 10 ppm wasserfreie Soda (4,3 ppm Na$^+$) versetzt. Nach 7stündigem Erhitzen auf 270 °C unter Rühren hatte sich der NCh-Gehalt auf 4 ppm erniedrigt. Die Destillation des Phthalsäureanhydrids ergab nun eine weitere Reduktion der NCh-Konzentration auf 0,5 ppm.

### Beispiel 8

Rohphthalsäureanhydrid (Gehalt: 0,3 Gew.-% MSA und 0,04 Gew.-% NCh) wurde mit 0,01 Gew.-% CDT sowie 25 ppm wasserfreie Soda (11 ppm Na⁺) versetzt und unter Rühren 7 h auf 270 °C erhitzt. Nach dieser Behandlung war der NCh-Gehalt auf weniger als 5 ppm gefallen. Die Feindestillation ergab ein Reinphthalsäureanhydrid von 1,0 ppm NCh.

### Beispiel 9

500 g Rohphthalsäureanhydrid mit einem Gehalt an NCh und MSA von je 2 Gew.-% wurden 7 h bei 270 °C mit 0,1 Gew.-% CDT und 30 ppm wasserfreiem $Na_2CO_3$ (13 ppm Na⁺) raffiniert. Durch die Behandlung wurde der Naphthochinon-Gehalt auf 5 ppm gesenkt, und nach der anschliessenden Destillation betrug der Gehalt an Naphthochinon im Reinprodukt 0,5 ppm.

### Beispiel 10

Ein Rohphthalsäureanhydrid der gleichen Zusammensetzung wie im Beispiel 9 wurde bei 280 °C unter Rühren 7 h mit 1,0 Gew.-% CDT und 5 ppm Ätzkali (3,5 ppm K⁺) behandelt. Auf diese Weise konnte der NCh-Gehalt auf 5 ppm reduziert werden. Nach der Destillation des Rohphthalsäureanhydrids betrug die NCh-Konzentration 0,5 ppm.

### Beispiel 11

500 g Rohphthalsäureanhydrid, das 0,6 Gew.-% NCh und 0,3 Gew.-% MSA enthielt, wurden mit 0,2 Gew.-% CDT und 30 ppm wasserfreiem $Na_2CO_3$ (13 ppm Na⁺) bei 270 °C 4 h lang behandelt. Der NCh-Gehalt sank auf 2 ppm und nach der Destillation des Raffinats lag er bei 1 ppm.

### Beispiel 12

Ein Rohphthalsäureanhydrid der gleichen Zusammensetzung wie im Beispiel 11 wurde mit 0,2 Gew.-% CDT sowie 30 ppm wasserfreiem $K_2CO_3$ (16,9 ppm K⁺) bei 270 °C 4 h mit gleichem Erfolg wie beim Beispiel 11 angegeben raffiniert.

### Beispiel 13

Das im Beispiel 11 beschriebene Rohphthalsäureanhydrid wurde 7 h bei 270 °C ausschliesslich mit 0,6 Gew.-% CDT behandelt. Danach war der NCh-Gehalt auf 2 ppm gefallen. Die anschliessende Feindestillation ergab ein Reinphthalsäureanhydrid mit einem NCh-Restgehalt von 1 ppm.

### Beispiel 14

500 g Rohphthalsäureanhydrid mit einem Gehalt an NCh von 1 Gew.-% und an MSA von 0,5 Gew.-% wurde mit 0,2 Gew.-% CDT und 30 ppm wasserfreiem $Na_2CO_3$ (13 ppm Na') 7 h lang bei 220 °C raffiniert. Der Naphthochinon-Gehalt wurde auf 1 ppm gesenkt. Das destillierte Reinprodukt wies ebenfalls einen Naphthochinon-Gehalt von 1 ppm auf.

### Beispiel 15

Ein Rohphthalsäureanhydrid mit 0,5 Gew.-% MSA und 0,8 Gew.-% NCh wurde mit 0,2 Gew.-% CDT und 3 ppm wasserfreiem $Na_2CO_3$ (1,3 ppm Na⁺) versetzt. Nach 7stündigem Erhitzen auf 240 °C war der Gehalt an NCh auf 3 ppm abgesunken. Die Feindestillation ergab eine Verbesserung auf 1 ppm NCh.

### Beispiel 16

500 g Rohphthalsäureanhydrid, dessen Gehalt an NCh 0,5 Gew.-% und an MSA 0,04 Gew.-% betrug, wurden bei 270 °C 7 h lang mit 0,1 Gew.-% Destillationsrückstand der CDT-Destillation behandelt. Das Gemisch hatte folgende Zusammensetzung:

| | | | |
|---|---|---|---|
| CDT | : 18 | Gew.-% | |
| $C_{16}$ | : 38 | Gew.-% | Butadien-Oligomere |
| $C_{20}$ | : 31 | Gew.-% | Butadien-Oligomere |
| $C_{24}$ | : 8 | Gew.-% | Butadien-Oligomere |
| $C_{28}$ | : 4 | Gew.-% | Butadien-Oligomere |
| $C_{32}$ | : 1,5 | Gew.-% | Butadien-Oligomere |

Das raffinierte Produkt hatte einen Naphthochinon-Gehalt von 1 ppm und das destillierte von 0,5 ppm.

### Beispiel 17

Beispiel 16 wurde wiederholt mit dem Unterschied, dass dem Rohprodukt zusätzlich noch 30 ppm wasserfreies $Na_2CO_3$ (13 ppm Na') hinzugegeben wurden. Der Naphthochinon-Gehalt im destillierten Reinprodukt war < 0,5 ppm.

### Beispiel 18

Beispiel 16 wurde wiederholt mit der Variation, dass zu dem Rohphthalsäureanhydrid 0,2 Gew.-% Destillationsrückstand der dort angeführten Zusammensetzung und 25 ppm Lithiumcarbonat (4,7 ppm Li⁺) gefügt wurde. Nach der Reindestillation wurde noch ein NCh-Gehalt von 0,5 ppm festgestellt.

### Beispiel 19

Beispiel 16 wurde wiederholt, jedoch wurde in diesem Beispiel ein Rohprodukt eingesetzt, dessen NCh-Gehalt 2 Gew.-% und dessen Gehalt an MSA 0,01 Gew.-% betrug. An Raffinationsmittel wurden 0,3 Gew.-% des im Beispiel 16 aufgeführten Destillationsrückstandes und 30 ppm wasserfreies $Na_2CO_3$ (13 ppm Na ') hinzugegeben. Nach der Behandlung betrug der Naphthochinon-Gehalt 3 ppm und nach der Destillation 1 ppm.

### Beispiel 20

Beispiel 16 wurde mit einem Rohprodukt wiederholt, dass 0,01 Gew.-% MSA und 1,3 Gew.-% NCh enthielt. Als Raffinationsmittel kam 0,7 Gew.-% des im Beispiel 16 beschriebenen Destillationsrückstandes sowie 40 ppm Lithiumhydroxid (11,6 ppm Li⁺) zum Einsatz. Nach der Destillation konnte ein Reinphthalsäureanhydrid erhalten werden, das nur noch 0,5 ppm NCh enthielt.

### Patentansprüche

1. Verfahren zur Entfernung von Naphthochinon aus durch Luftoxidation von Naphthalin oder Naphthalin enthaltenden Gemischen hergestelltem Phthalsäureanhydrid, dadurch gekennzeichnet, dass das Rohphthalsäureanhydrid mit 1,5,9-Cyclododecatrien und/oder dessen Destillationsrückstand bei höheren Temperaturen behandelt wird.

2. Verfahren nach Anspruch 1, dadurch gekennzeichnet, dass die Menge an Cyclododecatrien und/oder dessen Destillationsrückstand, bezogen auf Rohphthalsäureanhydrid 0,01 bis 1 Gew.-%, vorzugsweise 0,05 bis 0,5 Gew.-% beträgt.

3. Verfahren nach den Ansprüchen 1 und 2, dadurch gekennzeichnet, dass die Behandlung des Rohphthalsäureanhydrids bei einer Temperatur von 180 °C bis 280 °C für 4 bis 20 h durchgeführt wird.

4. Verfahren nach den Ansprüchen 1 bis 3, dadurch gekennzeichnet, dass das raffinierte Phthalsäureanhydrid destilliert wird.

5. Verfahren nach den Ansprüchen 1 bis 4, dadurch gekennzeichnet, dass das Rohphthalsäureanhydrid zusätzlich mit Alkaliionen behandelt wird.

6. Verfahren nach Anspruch 5, dadurch gekennzeichnet, dass die Menge an Alkaliionen 1 – 40 ppm, vorzugsweise 2 – 20 ppm, bezogen auf Rohphthalsäureanhydrid, beträgt.

## Revendications

1. Procédé d'élimination de naphtoquinone dans l'anhydride d'acide phtalique préparé par oxydation par l'air de naphtalène ou de mélanges contenant du naphtalène, caractérisé en ce que l'anhydride d'acide phtalique brut est traité à haute température avec du 1, 5, 9-cyclododécatriène et/ou son résidu de distillation.

2. Procédé selon la revendication 1, caractérisé en ce que la quantité de cyclododécatriène et/ou de son résidu de distillation, par rapport à l'anhydride d'acide phtalique brut est comprise entre 0,01 et 1% en poids, de préférence 0,05 à 0,5% en poids.

3. Procédé selon les revendications 1 et 2, caractérisé en ce que le traitement de l'anhydride d'acide phtalique brut est réalisé à une température comprise entre 180 °C et 280 °C pendant 4 à 20 h.

4. Procédé selon les revendications 1 à 3, caractérisé en ce que l'anhydride d'acide phtalique raffiné est distillé.

5. Procédé selon les revendications 1 à 4, caractérisé en ce que l'anhydride d'acide phtalique brut est en plus traité avec des ions alcalins.

6. Procédé selon la revendication 5, caractérisé en ce que la quantité en ions alcalins est de 1 – 40 ppm, de préférence 2 – 20 ppm, par rapport à l'anhydride d'acide phtalique brut.

## Claims

1. A process for removal of naphthaquinone from phthalic anhydride produced by air oxidation of naphthalene or of mixtures which contain naphthalene, characterized in that the crude phthalic anhydride is treated with 1,5,9-cyclododecatriene and/or its distillation residue at increased temperatures.

2. A process according to Claim 1, characterized in that the amount of cyclododecatriene and/or its distillation residue is 0,01 to 1% by weight, and preferably 0,05 to 0,5% by weight, relative to the crude phthalic anhydride.

3. A process according to Claim 1 and 2, characterized in that the treatment of the crude phthalic anhydride is performed at a temperature of 180 °C to 280 °C for 4 to 20 h.

4. A process according to Claims 1 to 3, characterized in that the refined phthalic anhydride is distilled.

5. A process according to Claims 1 to 4, characterized in that the crude phthalic anhydride is additionally treated with alkali ions.

6. A process according to Claim 5, characterized in that the amount of alkali ions is 1 – 40 ppm, and preferably 2 – 20 ppm, relative to the crude phthalic anhydride.